# EUROPÄISCHE PATENTSCHRIFT

(11) **EP 1 762 955 B1**
(45) Veröffentlichungstag und Bekanntmachung des Hinweises auf die Patenterteilung: **12.07.2017**
(21) Anmeldenummer: 06017111.3
(22) Anmeldetag: 17.08.2006
(51) Int. Cl.: G06F 19/00

(54) **Kommunikations-Adapter für ambulante medizinische oder therapeutische Geräte**
Communication adaptor for portable medical or therapeutical devices
Adaptateur de communications pour appareil médical ou thérapeutique

(30) Priorität: 27.08.2005 EP 05018651
(43) Veröffentlichungstag der Anmeldung: 14.03.2007
(73) Patentinhaber: Roche Diabetes Care GmbH, 68305 Mannheim (DE); F. Hoffmann-La Roche AG, 4070 Basel (CH)
(72) Erfinder: Koehler, Matthias, 69514 Laudenbach (DE); Blasberg, Peter, 69469 Weinheim (DE); Handwerker, Guenter, 67459 Böhl-Iggelheim (DE); Aigner, Manfred, 85551 Heimstetten (DE); Habermann, Christian, 85778 Haimhausen (DE)
(74) Vertreter: Meinel, Anne Julia

(56) Entgegenhaltungen:
- WO-A-2005/009514
- WO-A-2005/083619
- WO-A2-00/18449
- US-A1- 2001 056 226
- US-B1- 6 558 321
- US-B1- 6 584 336
- RICK MERRIT: "Wireless Hospital, Health Care Products On The Upswing" INTERNET CITATION, [Online] 7. Januar 2004 (2004-01-07), XP002348225 Gefunden im Internet: URL:http://www.techweb.com/article/printab leArticleSrc.jhtml?articleID=26803705> [gefunden am 2005-10-07]
- STEINFELD E F: "INTERNET-APPLIANCE TECHNOLOGY AUTOMATES TEST EQUIPMENT" EDN ELECTRICAL DESIGN NEWS, REED BUSINESS INFORMATION, HIGHLANDS RANCH, CO, US, Bd. 45, Nr. 21, 12. Oktober 2000 (2000-10-12), Seiten 157-158,160,16, XP001143050 ISSN: 0012-7515

## Beschreibung

Die Erfindung betrifft einen Kommunikations-Adapter zur Verwendung mit einem tragbaren, ambulant betreibbaren medizinischen oder therapeutischen Gerät, insbesondere einem Gerät für die Diagnose oder Behandlung einer Glukosestoffwechselstörung. Ein solcher Kommunikations-Adapter dient zum Übertragen von Daten zwischen dem medizinischen oder therapeutischen Gerät und einem Computer, der zum Anzeigen von Betriebsparametern oder Messdaten des Geräts und/oder zum Bedienen des Geräts dient.

Ein solcher Kommunikations-Adapter wird zwischen das medizinische oder therapeutische Gerät und den Computer geschaltet und kann Daten von dem Gerät zu dem Computer übertragen, um Betriebsparameter oder Messdaten des Geräts mit Hilfe des Computers darzustellen. Wenn der Kommunikations-Adapter auch zum Übertragen von Daten von dem Computer zu dem medizinischen oder therapeutischen Gerät ausgebildet ist, kann das Gerät auch mittels des Computers bedient werden, beispielsweise um das Gerät zu konfigurieren oder bestimmte Aktionen des Geräts auszulösen.

Das medizinische oder therapeutische Gerät umfasst dementsprechend einen Geräteprozessor zum Steuern des Gerätes und eine Geräte-Adapter-Schnittstelle zur Kommunikation des Geräteprozessors mit dem Kommunikations-Adapter. Der Kommunikations-Adapter umfasst einen Adapterprozessor zum Steuern des Kommunikations-Adapters, eine Adapter-Geräte-Schnittstelle zur Kommunikation des Kommunikations-Adapters mit dem Gerät, eine Adapter-Computer-Schnittstelle zur Kommunikation des Adapterprozessors mit einer Computer-Schnittstelle des Computers und einen Gerätetreiber mit zugehörigem Übertragungsprotokoll für die Kommunikation mit dem Gerät.

Die Geräte, die mit einem erfindungsgemäßen Kommunikations-Adapter Daten mit einem Computer austauschen können, sind in der Regel netzunabhängig betreibbar. Die Erfindung richtet sich insbesondere auf Geräte für die Diagnose oder Behandlung einer Erkrankung, insbesondere einer Glukosestoffwechselstörung wie Diabetes. Sie richtet sich also auf analytische Messgeräte zum Bestimmen oder Aufzeichnen (so genannte "elektronische Tagebücher") eines medizinisch bedeutsamen Parameters, insbesondere ein Blutglukosemeter, ein Blutzuckermessgerät, ein Cholesterinmessgerät, ein Blutgerinnungsparameter-Messgerät, ein Blutdruckmessgerät, ein Gerät zum Messen des Körpergewichts, ein Blutglukoserecorder oder ein Blutdruckrecorder, oder auf Geräte zum Injizieren eines medizinischen Wirkstoffs in einen Körper, insbesondere eine Insulinpumpe. Besonders bevorzugt werden im Rahmen der Erfindung medizinische oder therapeutische Geräte benutzt, die eigenständig, d.h. unabhängig von anderen angeschlossenen Komponenten oder Geräten, d.h. insbesondere ohne den Kommunikations-Adapter funktionsfähig und betreibbar sind. Dies bedeutet beispielsweise, dass ein Messgerät Messwerte liefert oder eine Insulinpumpe arbeitet, ohne an den Kommunikations-Adapter oder ein anderes Geräts angeschlossen zu sein.

Ein Blutglukosemeter ist ein Blutglukose-Messgerät, mit dessen Hilfe der Blutzuckergehalt bestimmt werden kann. In der Regel wird hierzu in einem Körper eine Einstichwunde erzeugt, ein Bluttropfen entnommen und mit Hilfe des Blutzuckermessgeräts der Blutglukosegehalt in dem Tropfen bestimmt. Es ist aber auch denkbar, durch eine permanente Messung, beispielsweise mit in den Körper eingeführten Sensoren oder durch die Haut hindurch die Blutglukose zu messen.

Insulinpumpen sind kleine Infusionsgeräte, die am Körper getragen werden und die über einen Katheter und einer unter der Haut liegenden Nadel dem Körper kontinuierlich oder in Intervallen Insulin zuführen. Dabei kann die Dosierung auch einem bestimmten Tagesrhythmus oder bestimmten Ereignissen, wie beispielsweise der Einnahme von Mahlzeiten, angepasst werden.

Blutglukoserecorder sind Geräte, die über eine vorgegebene Zeitdauer Blutglukosekonzentrationen aufzeichnen, beispielsweise um für einen Diabetes-Patienten ein geeignetes Insulindosierungsschema festlegen zu können.

Im Folgenden wird die Erfindung ohne Beschränkung der Allgemeinheit anhand derartiger Geräte für die Diagnose oder Behandlung einer Glukosestoffwechselstörung beschrieben.

Aus dem Dokument WO 98/59487 ist ein Kommunikations-Adapter bekannt, der über eine Infrarot-Schnittstelle Daten von einem medizinischen Gerät empfängt und über das Internet an eine zentrale Datenbank sendet. Hierzu wird der Kommunikations-Adapter an die Tastaturschnittstelle eines Computers mit Internet-Anschluss angeschlossen. Der Kommunikations-Adapter wandelt die von dem Gerät empfangenen Daten in Tastenbefehle um, so dass der Computer die Daten an eine zentrale Datenbank sendet. Von der zentralen Datenbank werden die empfangenen Daten aufbereitet und an den Computer, an dem der Kommunikations-Adapter angeschlossen ist, zurückgesendet und mit Hilfe eines Internet-Browsers dargestellt. Nachteilig bei diesem System ist, dass der Benutzer die Tastatur von dem Computer trennen muss, so dass der Computer nicht mehr bedient werden kann, während der Kommunikations-Adapter angeschlossen ist. Ein weiterer Nachteil ist, dass die mittels des Kommunikations-Adapters aus dem Gerät ausgelesenen Daten nur mit Hilfe eines Computers dargestellt werden können, der über einen Internet-Anschluss verfügt, und auch nur dann, wenn die Internet-Verbindung tatsächlich besteht.

Aus dem Dokument WO 00/18449 A2 ist ein Gestell mit integrierten Infrarot-Ports bekannt, in das ein Blutglukosemeter oder eine Insulinpumpe eingelegt werden kann, um mit dem Gestell Daten auszutauschen. Dabei muss der Bediener über einen Schalter das jeweils zu dem eingelegten Gerät passende Kommunikationsprotokoll auswählen.

In dem Dokument US 6,564,105 B2 ist ein Kommunikationsprotokoll für ein implantiertes medizinisches Gerät, beispielsweise eine Infusionspumpe beschrieben.

Das Dokument US 2003/0163088 offenbart eine Infrarot-Schnittstelle in einem Computer oder in einer externen Station zum ausschließlichen Programmieren von Insulinpumpen.

Aus dem Dokument US 2003/0032867 ist bekannt, Blutglukosemesswerte zusammen mit einem Zeit- und einem Datums-Stempel in einem Protokoll zu speichern. Die dazu gehörigen Patientendaten werden in einem weiteren Protokoll gespeichert. Die getrennte Speicherung in verschiedenen Protokollen führt zu einem erhöhten Aufwand, wenn die Daten zusammengeführt werden, z.B. in Patientenakten im Krankenhaus oder in der Arztpraxis.

Aus der Patentfamilie zu der US provisional patent application No. 60/177,414, Medical Research Group Inc. (z.B. US 6,577,899 und US 6,635,014), ist ein medizinisches System mit einem ambulanten medizinischen Gerät wie einem Blutglukosemeter oder einer Insulinpumpe bekannt, bei dem das Gerät eine Telemetrie-Einheit aufweist, die mit einer Telemetrie-Einheit eines Kommunikationsgerätes zum Auslesen von Daten und zum Programmieren der Geräte kommuniziert.

Aus dem nach dem Prioritätstag der vorliegenden Anmeldung veröffentlichten Dokument WO 2005/083619 A2 ist ein Netzwerk zum Kommunizieren mit medizinischen Geräten bekannt, bei dem ein Kommunikations-Server verwendet wird. Zum Herstellen der Verbindung mit dem medizinischen Gerät ist ein Intranet oder das Internet erforderlich, und zum Steuern der Verbindung ist zusätzlich zu dem medizinischen Gerät ein weiteres "remote device" erforderlich.

Das Dokument US 2005/0113655 A1 bezieht sich auf ein Pulsoximeter-Gerät, bei dem ein Sensor die Messrohdaten mittels einer drahtlosen Übertragung an das Pulsoximeter-Gerät sendet, das Pulsoximeter-Gerät die Messrohdaten mittels eines Prozessors aufbereitet und diese dann drahtlos an einen Empfänger zum Darstellen in Form einer Webseite sendet. Jedes Pulsoximeter-Gerät benötigt dabei somit seine eigene Software zum Aufbereiten und Darstellen der Daten sowie seine eigenen, zugehörigen und in das Gerät integrierten Hardware-Komponenten zum Übertragen der Daten.

In dem Dokument US 6,584,336 B1 wird ein Kommunikations-Adapter (Docketing Station) zum Verbinden eines Pulsoximeter-Geräts mit einem Computer-Interface beschrieben. Die Anschlüsse des Kommunikations-Adapters sowohl zu dem Pulsoximeter-Gerät als auch zu dem Computer-Interface sind drahtgebunden, so dass die Form des Kommunikations-Adapters und die Position der Steckverbindungen darin auf die Kontur des einzulegenden Geräts abgestimmt sein müssen. Deshalb können nicht beliebig geformte, sondern nur bestimmte Geräte mit dem Kommunikations-Adapter ausgelesen werden.

Das Dokument WO 2005/009514 A2 offenbart ein Kommunikations-System zum Anschließen eines implantierbaren medizinischen Geräts an verschiedene periphere Einrichtungen mittels einer Programmiereinrichtung, die per drahtloser Datenübertragung mit dem medizinischen Gerät verbunden ist.

In dem Dokument US 6,558,321 B1 wird ein Kommunikations-Adapter (Docketing Device) zum Verbinden eines medizinischen Geräts mit einem Computer-Interface beschrieben. Die Datenübertragung zwischen dem medizinischen Gerät und dem Computer kann in beide Richtungen erfolgen.

Das Dokument US 2001/0056226 A1 beschreibt eine Aufzeichnungseinrichtung zum Aufzeichnen von Daten medizinischer Messgeräte und zum Übertragen der Daten über ein Netzwerk an einen Monitor.

Aus dem Dokument Rick Merrit, "Wireless Hospital, Health Care Products On The Upswing", 7. Januar 2004, XP002348225 ist ein Kommunikations-Adapter zur Verwendung mit einem tragbaren, ambulant betreibbaren medizinischen oder therapeutischen Gerät zum Übertragen von Daten zwischen dem medizinischen oder therapeutischen Gerät und einem Computer bekannt. Dabei umfasst der Kommunikations-Adapter einen Adapterprozessor zum Steuern des Kommunikations-Adapters, eine Adapter-Geräte-Schnittstelle zur Kommunikation des Kommunikations-Adapters mit dem Gerät, eine Adapter-Computer-Schnittstelle zur Kommunikation des Adapterprozessors mit einer Computer-Schnittstelle des Computers und einen Gerätetreiber mit zugehörigem Übertragungsprotokoll für die Kommunikation mit dem Gerät.

Ausgehend von diesem Stand der Technik besteht die der Erfindung zugrunde liegende Aufgabe darin, einen Kommunikations-Adapter zum Übertragen von Daten zwischen einem medizinischen oder therapeutischen Gerät und einem Computer zu schaffen, der vielseitiger einsetzbar und von dem Benutzer einfach bedienbar ist.

Diese Aufgabe wird durch einen Kommunikations-Adapter mit den Merkmalen den Anspruchs 1 gelöst. Bevorzugte Ausgestaltungen und Weiterbildungen der Erfindung sind Gegenstand der weiteren unabhängigen Ansprüche und ergeben sich aus den abhängigen Ansprüchen sowie der nachfolgenden Beschreibung mit zugehörigen Zeichnungen.

Gemäß der Erfindung wird ein Kommunikations-Adapter zur Verwendung mit einem tragbaren, ambulant betreibbaren medizinischen oder therapeutischen Gerät, insbesondere einem Gerät für die Diagnose oder Behandlung einer Glukosestoffwechselstörung, zum Übertragen von Daten zwischen dem medizinischen oder therapeutischen Gerät und einem Computer, der zum Anzeigen von Betriebsparametern oder Messdaten des Geräts und/oder zum Bedienen des Geräts dient,
wobei das medizinische oder therapeutische Gerät umfasst
- einen Geräteprozessor zum Steuern des Gerätes und
- eine Geräte-Adapter-Schnittstelle zur Kommunikation des Geräteprozessors mit dem Kommunikations-Adapter,
und wobei der Kommunikations-Adapter umfasst
- einen Adapterprozessor zum Steuern des Kommunikations-Adapters,
- eine Adapter-Geräte-Schnittstelle zur Kommunikation des Kommunikations-Adapters mit dem Gerät,
- eine Adapter-Computer-Schnittstelle zur Kommunikation des Adapterprozessors mit einer Computer-Schnittstelle des Computers und
- einen Gerätetreiber mit zugehörigem Übertragungsprotokoll für die Kommunikation mit dem Gerät,
vorgeschlagen, der die Besonderheit aufweist, dass
der Kommunikations-Adapter derart ausgebildet ist, dass er von dem Computer aus dadurch steuerbar ist, dass von dem Computer Lesebefehle an den Kommunikations-Adapter gesendet werden, durch die in dem Kommunikations-Adapter Triggerdateien gelesen werden, und das Lesen einer solchen Triggerdatei von der Soft- oder Firmware des Kommunikations-Adapters als Steuerbefehle für den Kommunikations-Adapter interpretiert wird.

Das Senden der Lesebefehle von dem Computer an den Kommunikations-Adapter erfolgt dabei bevorzugt über die Computer-Schnittstelle des Computers an die Adapter-Computer-Schnittstelle des Kommunikations-Adapters.

Die Befehlssteuerung des Kommunikations-Adapters erfolgt über Lesebefehle (Links) auf das Speichermedium des Kommunikations-Adapters. Wenn auf dem Bildschirm ein Link angeklickt wird, werden auf dem Kommunikations-Adapter ein oder mehrere bestimmte so genannte Triggerdateien gelesen und das Lesen einer solchen Datei wird von der Soft- oder Firmware des Kommunikations-Adapters als ein bestimmter Befehl, z.B. "Drucken" oder "Gerät lesen" interpretiert. Auf diese Weise ist es möglich, den Kommunikations-Adapter mit dem Computer zu steuern, und zwar mit einer standardmäßig auf dem Computer installierten Software wie beispielsweise einem Internet-Browser, ohne dass hierzu ein besonderes Software-Programm auf dem Computer installiert ist, das Befehle ausführen kann.

In dem bevorzugten Fall der Verwendung eines Internet-Browsers zum Darstellen der ausgelesenen Daten und zum Steuern des Kommunikations-Adapters ist ferner zu berücksichtigen, dass mit standardmäßigen Internet-Browsern keine Schreibbefehle an einen Computer gesendet werden können, um zu verhindern, dass beispielsweise beim Öffnen einer Internetseite durch solche Befehle das automatische Kopieren oder Löschen auf dem Computer vorhandener Dateien gestartet wird. Daher ist insbesondere bei der Verwendung von Internet-Browsern das Lesen von Triggerdateien zum Steuern des Kommunikations-Adapters von dem Computer aus vorteilhaft.

In der Praxis ist es vorteilhaft, wenn der Trigger für einen Befehl in dem Lesen mehrerer Dateien, bevorzugt in unterschiedlichen Verzeichnissen auf dem Kommunikations-Adapter, gleichzeitig oder in einer festen Reihenfolge nacheinander besteht. Auf diese Weise kann nicht nur der Codierungsgrad für die möglichen Befehle, sondern auch die Sicherheit erhöht werden, beispielsweise um zu vermeiden, dass beim Scannen des Kommunikations-Adapters mit einem Virenscanner oder beim Betrachten des Inhalts des Kommunikations-Adapters mit einem anderen Programm, beispielsweise einem Windows-Explorer, unbeabsichtigt Befehle an den Kommunikations-Adapter gesendet werden.

Ein erfindungsgemäßer Kommunikations-Adapter kann ferner vorteilhafterweise die Besonderheit aufweisen, dass der Kommunikations-Adapter die erforderliche Soft- oder Firmware umfasst, um die von dem Gerät ausgelesenen Daten unabhängig von dem Computer aufzubereiten und in einem von dem Computer mit einem Internet-Browser darstellbaren Datenformat zum Auslesen durch den Computer über die Adapter-Computer-Schnittstelle für die Darstellung der Daten an dem Computer bereitzustellen.

Wenn der Kommunikations-Adapter über die Fähigkeit, d.h. die erforderliche Soft- oder Firmware verfügt, um die Daten für die Darstellung mit einem Internet-Browser des Computers aufzubereiten, ist an dem Computer kein besonderes zusätzliches Programm zum Darstellen der Daten erforderlich. Das von dem Kommunikations-Adapter zur Darstellung der mit dem Computer von dem Gerät ausgelesenen Daten erzeugte Datenformat ist ein von einem Internet-Browser darstellbares Format.

Eine Soft- bzw. Firmware zum Auslesen der Geräte und zum Aufbereiten der Daten befindet sich in dem Kommunikations-Adapter. Dieser leitet die Daten in einem von einem Internet-Browser darstellbaren Format, z.B. als HTML-Datei an den Computer weiter, wenn der Computer die Daten aus dem Kommunikations-Adapter ausliest. In dem Computer wird ein Internet-Browser als standardmäßiges Programm verwendet, um die Daten in Form von Graphen, Statistiken und Tabellen auf dem Monitor des Computers anzuzeigen. Ein standardmäßiges Programm in diesem Sinne ist ein Programm, das auch auf einem Computer installiert wäre, der nicht zur Benutzung mit einem erfindungsgemäßen Kommunikations-Adapter vorgesehen ist. Bevorzugte standardmäßige Programme im Rahmen der Erfindung sind Internet-Browser wie z.B. Internet Explorer, Netscape oder Mozilla.

Ein solcher Internet-Browser ist in der Regel in der Standardsoftware eines Computers installiert, wobei die verschiedenen gebräuchlichen Internet-Browser aus Kompatibilitätsgründen derart ausgelegt sind, dass sie dasselbe Datenformat verarbeiten können, insbesondere ein HTML-Datenformat.

Der Kommunikations-Adapter dient als temporärer Speicher für die von dem Gerät empfangenen Daten bzw. HTML-Daten. Über Monitor, Maus und Tastatur des Computers wird der Kommunikations-Adapter zum Auslesen der Daten gesteuert. Der Computer dient also nur als User-Interface für das Anzeigen der Daten und das Bedienen des Kommunikations-Adapters, ohne dass hierzu eine spezielle Software auf dem Computer installiert oder gestartet werden muss. Aus Datenschutzgründen ist es vorteilhaft, wenn dabei keine der von dem Gerät bzw. dem Kommunikations-Adapter ausgelesenen Daten auf dem Computer gespeichert werden.

Die nachfolgend beschriebenen Merkmale sind vorteilhaft einsetzbar.

Nach einem ersten vorteilhaften Merkmal wird vorgeschlagen, dass die Adapter-Geräte-Schnittstelle zur Kommunikation des Kommunikations-Adapters mit dem Gerät eine Schnittstelle zur drahtlosen Datenübertragung, insbesondere eine Infrarot-Schnittstelle ist. Dementsprechend ist dann auch die Geräte-Adapter-Schnittstelle zur Kommunikation des Geräteprozessors mit dem Kommunikations-Adapter eine Schnittstelle zur drahtlosen Datenübertragung, insbesondere eine Infrarot-Schnittstelle.

Prinzipiell kann die Adapter-Geräte-Schnittstelle auf jede beliebige Weise implementiert sein. Im Falle einer drahtgebundenen Datenübermittlung muss der Benutzer des Gerätes jedoch ein passendes Anschlusskabel zur Verfügung haben und anschließen, um die Kommunikation des Gerätes mit einem Kommunikations-Adapter aufzubauen. Dies kann, insbesondere bei erkrankten Personen, Schwierigkeiten bereiten. Darüber hinaus können die Steckverbindungskontakte verschmutzen oder beschädigt werden. Die drahtlose Kommunikation zwischen dem Gerät und dem Kommunikations-Adapter, beispielsweise per Funk, ist daher bevorzugt. In der Praxis ist die Implementierung in Form einer Infrarot-Schnittstelle bevorzugt, da diese leicht benutzbar und unanfällig sowie darüber hinaus kostengünstig realisierbar ist, so dass die meisten Geräte ohnehin über eine solche Infrarot-Schnittstelle verfügen. Aber auch eine sich immer mehr verbreitende Bluetooth-Schnittstelle, die eine drahtlose Kommunikation per Funk ermöglicht, kann vorteilhaft sein.

Die Adapter-Computer-Schnittstelle zur Kommunikation des Adapterprozessors mit der Computer-Schnittstelle des Computers und dementsprechend die Computer-Schnittstelle des Computers ist dagegen bevorzugt eine Schnittstelle zur drahtgebundenen Datenübertragung. Bei gebräuchlichen Computern ist es unüblich, eine Schnittstelle für die drahtlose Kommunikation ab Werk vorzusehen, dagegen ist der Einbau von Schnittstellen für die drahtgebundene Kommunikation, beispielsweise einer USB-Schnittstelle, der Regelfall. Eine drahtlose Kommunikation zwischen Computer und Kommunikations-Adapter kann aber ebenfalls vorteilhaft sein, beispielsweise in Form einer WLAN-Verbindung.

Die Verwendung einer drahtgebundenen Schnittstelle für die Datenübertragung zwischen Kommunikations-Adapter und Computer vergrößert daher den Anwendungsbereich für einen Benutzer. Dabei ist es für den Benutzer in vielen Anwendungsfällen nicht nachteilig, dass eine Kabelverbindung zwischen Computer und Kommunikations-Adapter hergestellt werden muss, beispielsweise wenn der Kommunikations-Adapter nicht von dem Benutzer des Gerätes mitgeführt wird, beispielsweise wenn sich der Benutzer mit seinem Gerät an eine Auslesestation begibt, an der bereits ein Kommunikations-Adapter an einen Computer angeschlossen ist, wie z.B. in einer Apotheke oder in einer Arztpraxis.

Nach einem zusätzlichen bevorzugten Merkmal wird vorgeschlagen, dass der Kommunikations-Adapter eine Mehrzahl von Gerätetreibern mit zugehörigen Übertragungsprotokollen aufweist, die bei einem über die Adapter-Geräte-Schnittstelle mit dem Kommunikations-Adapter kommunizierenden Gerät automatisch abgefragt werden, um einen für das aktuelle Gerät passenden Gerätetreiber aus der Mehrzahl der Gerätetreiber zu wählen. Ein Kommunikations-Adapter kann dann als Zusatzgerät mit einer Mehrzahl von medizinischen oder therapeutischen Geräten oder mit Baureihen solcher Geräte verwendet werden. Die medizinischen oder therapeutischen Geräte selbst benötigen keine Hard- oder Softwarekomponenten, die die Funktionen des Kommunikations-Adapters bereitstellen, und können dadurch unaufwendiger hergestellt werden. Hierbei kann auch das Kommunikationsprotokoll zu verschiedenen Geräten unterschiedlich sein, solange die Voraussetzungen zur Nutzung derselben Hardware vorliegen. Beispielsweise können manche Geräte einen proprietären Kommunikations-Standard und andere einen allgemeinen IrDA-Standard verwenden.

Vorteilhafterweise wird das Durchprobieren der Gerätetreiber zum Aufbauen einer Kommunikation mit einem Gerät automatisch gestartet, sobald der Kommunikations-Adapter mit Strom versorgt wird. Das Durchprobieren kann auch beim Erkennen eines Gerätes fortgesetzt werden, um zu überprüfen, ob eventuell weitere Geräte vorhanden sind, mit denen ggf. gleichzeitig kommuniziert werden soll, oder das Durchprobieren kann beim Trennen einer Kommunikationsverbindung zu einem Gerät neu gestartet werden, um die Verbindung zu diesem oder einem anderen Gerät neu aufzubauen. Diese automatischen Abläufe erleichtern die Verwendung des Kommunikations-Adapters durch einen Benutzer oder insbesondere durch wechselnde Benutzer oder bei der Verwendung unterschiedlicher Geräte.

Auf diese Weise kann ein mit dem Kommunikations-Adapter auszulesendes Gerät, beispielsweise ein Blutglukosemeter, eine Insulinpumpe oder ein elektronisches Tagebuch für Blutglukosewerte, automatisch von dem Kommunikations-Adapter erkannt werden. Geräte wie die für die Diagnose oder Behandlung einer Glukosestoffwechselstörung besitzen zumeist eine Infrarot-Schnittstelle für die externe Kommunikation, die jeweils verwendeten Protokolle und Befehlssätze sind jedoch unterschiedlich. Daher ist es vorteilhaft, wenn die Soft- bzw. Firmware des Kommunikations-Adapters die in Frage kommenden Gerätetreiber, die die entsprechenden Befehlssätze beinhalten, aufweist. Während des Aufbaus der Kommunikation zwischen dem Kommunikations-Adapter und dem Gerät fragt der Kommunikations-Adapter nacheinander mit den in ihm vorhandenen Kommunikationsprotokollen (Treibern) das zunächst unbekannte Gerät an, bis er das passende Kommunikationsprotokoll gefunden hat, mit dem die Kommunikation mit dem Gerät durchführbar ist. Ab dann wird die Kommunikation mit diesem gefundenen Treiber durchgeführt.

Dabei kann vorteilhafterweise auch die Übertragungsrate (baud rate) optimiert werden. Hierzu fragt beispielsweise der Kommunikations-Adapter mit einer niedrigen Ausgangs- oder Default-Übertragungsrate bei dem Gerät an, welche Übertragungsrate das Gerät ermöglicht, und schaltet danach auf die höchste gemeinsame Übertragungsrate um.

Auf diese Weise ist es aber nicht nur möglich, ein zunächst unbekanntes Gerät zu erkennen, sondern es kann auch mit künftigen, neu entwickelten Geräten kommuniziert werden, vorausgesetzt, dass die gleiche Art der Kommunikation, beispielsweise eine Infrarot- oder Funk-, z.B. Bluetooth-Schnittstelle verwendet wird. Ggf. muss lediglich ein neuer Softwaretreiber in dem Kommunikations-Adapter aufgespielt werden. Ein derartiges Software-Update kann beispielsweise über einen Download von einer im Internet vom Hersteller bereitgestellten Treiberdatei oder mittels einer CD erfolgen, wenn der Kommunikations-Adapter an einen Computer angeschlossen ist. Der Kommunikations-Adapter ist also updatefähig für die Kommunikation mit neuen Geräten oder mit geänderter Gerätesoftware.

Beim Aktivieren der Kommunikation des Kommunikations-Adapters mit einem Gerät kann das Suchen nach dem passenden Gerätetreiber in dem Kommunikations-Adapter dadurch optimiert werden, dass zunächst die Kommunikation mit dem zuletzt als zutreffend erkannten Gerätetreiber oder einem Gerätetreiberpaar, bestehend beispielsweise aus Blutglukosemeter und Insulinpumpe, gestartet wird oder dieser zuletzt verwendete Treiber beim Durchprobieren der Gerätetreiber häufiger als die anderen zum Testen eines Kommunikationsaufbaus eingesetzt wird. Dadurch kann bei den gebräuchlichen Anwendungen eines Benutzers mit seinen Geräten die Kommunikation zwischen dem Kommunikations-Adapter und dem Gerät schneller aufgebaut werden.

Nach einem vorteilhaften zusätzlichen Merkmal kann vorgesehen sein, dass der Kommunikations-Adapter zusätzlich die von dem Gerät ausgelesenen Daten in einem abspeicherbaren Datenformat erzeugt und diese Daten in mindestens einer Datei in dem Kommunikations-Adapter abspeichert, von wo sie über die Adapter-Computer-Schnittstelle von dem Computer auslesbar sind. Der Kommunikations-Adapter kann auch in diesem Fall in einem Stand-Alone-Modus arbeiten, erzeugt die Daten jedoch nicht nur beispielsweise als HTML-Datei für die Darstellung mit einem Internet-Browser, sondern zusätzlich z.B. als XML-Datei, die dann in eine Datenbank oder Tabelle, beispielsweise Excel, importiert werden kann.

Auf dem Computer ist hierzu ein Internet-Browser als standardmäßige Software oder eine firmenspezifische Software gespeichert, die die Dateien importieren, weiterverarbeiten und daraus die Darstellung generieren oder in einer Datenbank ablegen kann. Der Kommunikations-Adapter generiert diese Dateien selbständig, obwohl er sich eigentlich dem Computer gegenüber wie ein passives Gerät wie z.B. ein USB-Gerät oder ein Massenspeichergerät, beispielsweise wie ein Memory-Stick, darstellt. Der Kommunikations-Adapter kann den Computer durch sich Ab- und wieder Anmelden zum Neuladen des Dateisystems des Kommunikations-Adapters veranlassen und der Kommunikations-Adapter dient zusammen mit der firmenspezifischen Software als "intelligentes Kabel" zum Aufbereiten der von dem Gerät ausgelesenen Daten.

Die Erfindung wird nachfolgend anhand in den Figuren dargestellter Ausführungsbeispiele näher erläutert. Die darin beschriebenen Besonderheiten können einzeln oder in Kombination miteinander eingesetzt werden, um bevorzugte Ausgestaltungen der Erfindung zu schaffen. Es zeigen:
- Fig. 1: einen Kommunikations-Adapter zum Auslesen von Daten von Geräten und das Steuern des Kommunikations-Adapters durch das Lesen von Triggerdateien, als Folge von Lesebefehlen die von einem Computer an den Kommunikations-Adapter gesendet werden,
- Fig. 2: einen Kommunikations-Adapter zum Auslesen von Daten von Geräten und zum Bedienen der Geräte von einem Computer aus und
- Fig. 3: einen schematischen Aufbau der Prozesssteuerung eines Kommunikations-Adapters mit zugehöriger Systemumgebung.

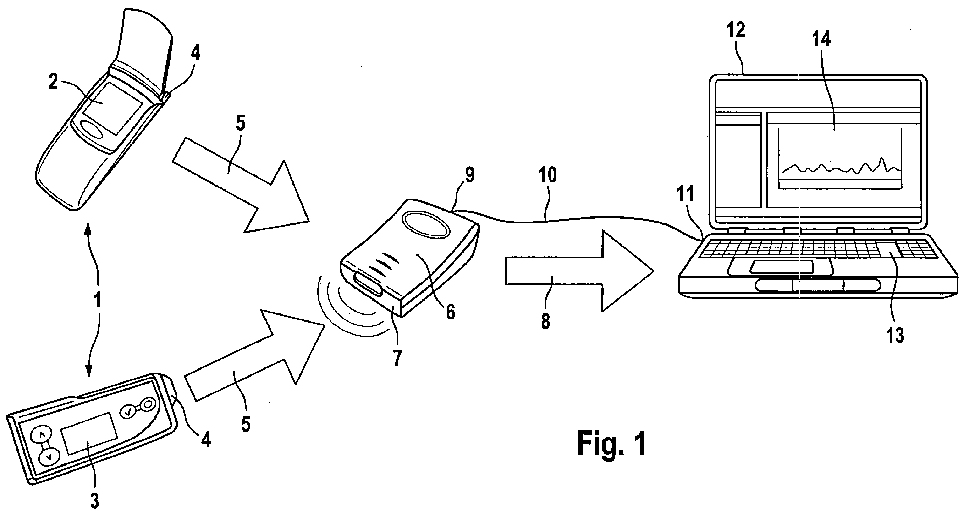

Die Fig. 1 zeigt zwei tragbare, netzunabhängig ambulant betreibbare medizinische Geräte 1, und zwar ein Blutglukosemeter 2 und eine Insulinpumpe 3, die von einem Benutzer ggf. in Kombination miteinander verwendet werden. Beide Geräte weisen eine Geräte-Adapter-Schnittstelle 4 zum Senden und/oder Empfangen von Daten auf, die als Infrarot-Schnittstelle ausgebildet ist. In dem in Fig. 1 dargestellten Ausführungsbeispiel erfolgt die Infrarot-Datenübertragung 5 durch Senden von Daten von der Geräte-Adapter-Schnittstelle 4 des Geräts 1 an einen Kommunikations-Adapter 6, der eine Adapter-Geräte-Schnittstelle 7 zum Senden und/oder Empfangen von Daten aufweist, die ebenfalls als Infrarot-Schnittstelle ausgebildet ist. Die Verbindung von dem Gerät 1 über die Geräte-Adapter-Schnittstelle 4 zu der Adapter-Geräte-Schnittstelle 7 des Kommunikations-Adapters 6 erfolgt vorzugsweise durch eine direkte kabelgestützte oder direkte drahtlose (Funk oder Infrarot) Verbindung und nicht über ein Netzwerk oder einen Server bzw. Web-Server (Intranet oder Internet), um die einfache, sichere und direkte Kommunikation zu gewährleisten.

Der Kommunikations-Adapter 6 kann vorteilhafterweise als Stand-Alone-Gerät ausgebildet sein. Er ist für die drahtgebundene Datenübertragung 8 mit einer Adapter-Computer-Schnittstelle 9 und einem Übertragungskabel 10 an eine Computer-Schnittstelle 11 eines Computers 12 angeschlossen. Der Computer 12 kann beispielsweise ein Personal-Computer, ein Laptop, ein Handheld-Computer oder der Computer in einem Internet-Cafe, in einer Arztpraxis oder in einer Apotheke, wohin sich der Benutzer der Geräte 1 nur mit seinem Gerät 1 oder mit seinem Gerät 1 und einem Kommunikations-Adapter 6 begibt, sein. Wenn der Benutzer eine Einrichtung aufsucht, an der ein Computer 12 mit einer einem Internet-Browser als Standard-Darstellungs-Software und einem für viele medizinische oder therapeutische Geräte-Typen ausgelegten Kommunikations-Adapter 6 vorhanden ist, beispielsweise eine entsprechend eingerichtete Apotheke, muss er selbst keinen Computer 12 oder Kommunikations-Adapter 6 mit sich führen, um die in seinem Gerät gespeicherten Daten darzustellen. Wenn der Benutzer einen Kommunikations-Adapter 6 mit sich führt, kann er seine Daten auch an beliebigen Computern 12 darstellen, die einen Internet-Browser als Standard-Darstellungs-Software aufweisen, beispielsweise in einem Internet-Cafe.

Eine drahtgebundene Computer-Schnittstelle 11 des Computers 12 kann beispielsweise eine serielle Schnittstelle, eine parallele Schnittstelle, eine Firewire-Schnittstelle oder bevorzugt eine USB-Schnittstelle sein. Das Übertragungskabel 10 und eine Adapter-Computer-Schnittstelle 9 an dem Kommunikations-Adapter 6 werden passend dazu ausgewählt. Ggf. kann ein Kommunikations-Adapter 6 mehrere drahtgebundene Schnittstellen in verschiedenen Standards aufweisen. Die USB-Schnittstelle ist bevorzugt, weil sie sehr weit verbreitet ist, einen geringen Platzbedarf hat und eine schnelle Datenübertragung ermöglicht. Auch eine drahtlose Kommunikation zwischen Kommunikations-Adapter 6 und Computer 12 kann vorteilhaft sein, beispielsweise in Form einer WLAN-Verbindung.

Zum Auslesen der Daten der Geräte 1 und deren Darstellung auf dem Monitor 14 des Computers 12 wird zunächst der Kommunikations-Adapter 6 mit dem Kabel 10 an den Computer 12 angeschlossen. In bevorzugten Ausführungsformen weist der Kommunikations-Adapter 6 keine eigene oder interne Stromquelle auf und seine Stromversorgung erfolgt über das Kabel 10 von der Computer-Schnittstelle 11 des Computers 12. Der Vorteil für den Benutzer besteht darin, dass der Kommunikations-Adapter 6 klein und leicht sein kann, da kein eigenes Netzteil erforderlich ist, und auch keine den Platzbedarf und das Gewicht erhöhenden Akkus oder Batterien enthalten sein und mitgeführt werden müssen.

Mit dem Anschließen des Kommunikations-Adapters 6 an den Computer 12 geht der Kommunikations-Adapter 6 automatisch in den Suchmodus über, in dem er nach Geräten 1 sucht, die mit seiner Adapter-Geräte-Schnittstelle 7 kommunizieren können. Alternativ kann auch vorgesehen sein, dass der Kommunikations-Adapter 6 durch eine Aktion des Benutzers, beispielsweise das Drücken einer Taste, in den Suchmodus geschaltet wird.

Wenn die Geräte-Adapter-Schnittstelle 4 des Gerätes 1 eine Infrarot-Schnittstelle ist, sollte sie mit der Adapter-Geräte-Schnittstelle 7 des Kommunikations-Adapters 6, die dann ebenfalls eine Infrarot-Schnittstelle ist, ausgerichtet werden, um die optische Datenübertragung zu ermöglichen. Der Kommunikations-Adapter 6 scrollt die in ihm gespeicherten Gerätetreiber durch und sucht nach einem passenden Gerät 1, das mit ihm kommunizieren kann.

Die Geräte-Adapter-Schnittstelle 4 an dem Gerät 1, insbesondere eine Infrarot-Schnittstelle, wird vorteilhafterweise durch eine manuelle Betätigung des Benutzers an dem Gerät 1 aktiviert, um die Kommunikation mit dem Kommunikations-Adapter 6 aufzubauen und anschließend die Datenübertragung bzw. Infrarot-Datenübertragung 5 zu ermöglichen. Die manuelle Aktivierung der Geräte-Adapter-Schnittstelle 4 ermöglicht es, dass sie beim sonstigen Gebrauch des Geräts 1 aus Stromspar- und Datensicherheitsgründen abgeschaltet ist.

Wenn der Computer 12 den angeschlossenen Kommunikations-Adapter 6 erkannt hat und die Kommunikation des Kommunikations-Adapters 6 mit dem Gerät 1 aufgebaut ist, kann der Benutzer an der Tastatur 13 des Computers einen Internet-Browser aufrufen und die von dem Gerät 1 ausgelesenen Daten mittels des Internet-Browsers auf dem Monitor 14 darstellen, ohne dass hierzu eine besondere, spezielle oder individuelle Programmierung des Computers 12 erforderlich ist. Der Benutzer des Gerätes 1 und des Kommunikations-Adapters 6 kann daher die Daten auf jedem Computer 12, auf dem ein Internet-Browser installiert ist, darstellen und betrachten, ohne hierzu eine spezielle Software für das Auslesen des Kommunikations-Adapters 6 auf dem Computer 12 installieren zu müssen.

Dies wird dadurch ermöglicht, dass der Kommunikations-Adapter 6 die erforderliche Soft- oder Firmware umfasst, um die von dem Gerät 1 ausgelesenen Daten unabhängig von dem Computer 12 aufzubereiten und in einem von dem Computer 12 mit einem Internet-Browser als standardmäßiger Darstellungs-Software darstellbaren Datenformat über die Adapter-Computer-Schnittstelle 9 an den Computer 12 zu senden bzw. zum Auslesen durch den Computer 12 bereitzustellen.

Ein anderer, selbständiger oder zusätzlicher erfindungsgemäßer Aspekt ist ebenfalls in Fig. 1 veranschaulicht, gemäß dem die Steuerung des Kommunikations-Adapters 6 von dem Computer 12 aus dadurch erfolgt, dass von dem Computer 12 Lesebefehle an den Kommunikations-Adapter 6 gesendet werden, durch die in dem Kommunikations-Adapter 6 Triggerdateien gelesen werden, die von der Soft- oder Firmware des Kommunikations-Adapters 6 als Steuerbefehle zum Steuern des Kommunikations-Adapters 6 interpretiert werden.

Da sich die zum Darstellen der Daten erforderliche Soft- bzw. Firmware in dem Kommunikations-Adapter 6 befindet und der Computer 12 nur als Anzeige und Bedienelement dient, der nur einen Internet-Browser als standardmäßige Darstellungs-Software benötigt, d.h. eine Darstellungs-Software, die zu den Standard-Programmen gehört, die gewöhnlich auf einem Computer installiert sind und somit einen hohen Verbreitungsgrad aufweist, ist die Verwendung des Kommunikations-Adapters 6 zum Darstellen der Daten unabhängig davon, welcher Computer 12 verwendet wird. Eine solche Standard-Darstellungs-Software kann beispielsweise ein Programm sein, das üblicherweise als Bestandteil der oder mit der Betriebssystemsoftware eines Computers an die Nutzer ausgeliefert wird, oder eine Darstellungs-Software, die einen hohen Verbreitungsgrad aufweist, beispielsweise ein Internet-Browser. Dadurch kann der Kommunikations-Adapter 6 von dem Benutzer sehr mobil genutzt werden, da der Kommunikations-Adapter 6 beispielsweise auch auf Reisen an einen beliebigen Computer 12 anschließbar ist. Es handelt sich um ein Real Plug & Play System; man muss lediglich die Verbindung zwischen Kommunikations-Adapter 6 und Computer 12, beispielsweise eine USB-Verbindung mit dem Übertragungskabel 10, herstellen und kann die Daten der Geräte 1 auf dem Computer 12 darstellen. Hierzu sind auch keinerlei Administratorrechte notwendig, so dass der Kommunikations-Adapter 6 selbst bei Rechnern verwendet werden kann, die gegen die Installation von zusätzlicher Software durch einen Benutzer gesperrt sind.

Ein weiterer Vorteil ist, dass es sich um ein so genanntes "Embedded System" handelt, das sehr virensicher ist. Da der Computer 12 nur als User-Interface dient und der Benutzer mit diesem User-Interface vertraut ist, da er die Bedienung eines Internet-Browsers, einer Tastatur, einer Maus und eines Monitors kennt, ist die Bedienung für den Benutzer sehr leicht erlernbar.

Die Verwendung des Computers 12 zum Darstellen der von den Geräten 1 ausgelesenen Daten hat für den Benutzer gegenüber einem Betrachten der Daten auf den Anzeigeelementen der Geräte 1 viele Vorteile. Auf den kompakten Geräten 1 befinden sich nur kleine Anzeigeelemente, die nur wenig Information in kleiner Auflösung, in kleinem Format und meist nur Einzelwerte darstellen können. Eine anspruchsvollere Darstellung oder Auswertung der Daten ist nicht oder nur sehr mühsam möglich. Auf dem Monitor 14 des Computers 12 kann dagegen eine aufwendige und aufbereitete Darstellung von Daten in Graphen oder Listen sowie eine Trendanalyse erfolgen. Allgemein kann es deshalb vorteilhaft sein, wenn das Gerät 1 und der Kommunikations-Adapter 6 zum Übertragen von Betriebsdaten oder Messwerten des Geräts 1 von dem Gerät 1 mittels des Kommunikations-Adapters 6 an den Computer 12 ausgebildet sind, bei denen es sich um Betriebsdaten oder Messwerte handelt, die sich auf zurückliegende Zeitpunkte beziehen. In diesem Fall werden also nicht oder nicht nur aktuelle Daten, sondern beispielsweise gespeicherte Messwerte, also Historien an den Computer 12 übertragen und dort dargestellt. Insbesondere bei der Diagnose oder Behandlung einer Glukosestoffwechselstörung ist der zurückliegende Verlauf von Betriebsdaten und Messwerten von medizinischem und therapeutischem Interesse.

Dabei kann der Benutzer sogar auch in Fällen, in denen die Datenübertragung, beispielsweise die Infrarot-Datenübertragung 5, nur von dem Gerät 1 zu dem Kommunikations-Adapter 6 und nicht von dem Kommunikations-Adapter 6 zu dem Gerät 1 erfolgt, die Darstellung auf dem Monitor 14 anpassen. Hierzu können in dem Kommunikations-Adapter 6 verschiedene, fest vorgegebene Darstellungsvarianten abgespeichert sein, die der Benutzer an dem Computer 12 alternativ abrufen kann.

Der Computer 12 kann jedoch nicht nur beispielsweise Messdaten, Profile, Muster, Trends, Fehlverhalten und Fehldosierungen graphisch darstellen, sondern auch Geräteparameter wie beispielsweise die Seriennummer des ausgelesenen Geräts 1 oder eine Patientenidentifikation. Die ausgelesenen HTML-Daten werden, anders als ggf. die mit einer anderen Software zusätzlich oder alternativ ausgelesenen XML-Daten, vorzugsweise im Standardfall nur auf dem Kommunikations-Adapter 6 gespeichert und beim Trennen der Adapter-Computer-Schnittstelle 9 von der Computer-Schnittstelle 11 auf dem Kommunikations-Adapter 6 gelöscht, was insbesondere bei Verwendung des Kommunikations-Adapters 6 an öffentlich zugänglichen Computern 12, beispielsweise in einer Arztpraxis oder einer Apotheke, in die sich ein Benutzer mit seinem Gerät 1 begibt, um an einem dort vorhandenen Kommunikations-Adapter 6 die Daten auszulesen, die Vertraulichkeit wahrt. Es besteht aber auch die Möglichkeit, an dem Kommunikations-Adapter 6 eine Reset-Taste oder auf dem Monitor 14 zur Bedienung des Kommunikations-Adapters 6 ein Reset-Icon vorzusehen, um die Daten auf dem Kommunikations-Adapter 6 manuell zu löschen.

Aus Gründen der Vertraulichkeit ist es ebenfalls vorteilhaft, wenn die von dem Gerät 1 ausgelesenen Daten in dem Kommunikations-Adapter 6 in einem flüchtigen Speicher gespeichert oder beim Trennen der Datenübertragungsverbindung zu einem Gerät 1, beim Anschließen eines neuen Gerätes 1 an den Kommunikations-Adapter 6, durch Drücken einer Reset-Taste an dem Kommunikations-Adapter 6 oder durch Anklicken eines Reset-Buttons auf dem Monitor 14 gelöscht werden.

Gemäß einem weiteren vorteilhaften Merkmal kann aus Gründen der Sicherheit und Vertraulichkeit vorgesehen sein, dass die Darstellungs-Software eine Software ist, die die mit dem Computer 12 darzustellenden Daten zwar von dem Kommunikations-Adapter 6 lesen, aber nicht auf dem Kommunikations-Adapter 6 bzw. in dem Gerät 1 ändern kann, so dass die mit dem Computer 12 von dem Gerät 1 ausgelesenen Daten nur zur temporären Darstellung an den Computer 12 übertragen werden, indem die Darstellungs-Software die Daten von dem Gerät 1 ausliest, die Darstellungs-Software aber die dabei auf dem Kommunikations-Adapter 6 verbleibenden Daten auf dem Kommunikations-Adapter 6 bzw. die auf dem Gerät 1 verbleibenden Daten auf dem Gerät 1 nicht löschen oder verändern kann.

Die Daten werden in diesem Fall also nicht auf dem Computer 12 gespeichert, sondern von dem Computer 12 mit dem Internet-Browser als Standard-Darstellungs-Software nur temporär zur Darstellung gebracht. Dies ist ähnlich zu dem Öffnen einer Textdatei mit einem Textbearbeitungsprogramm, wobei die Textdatei zwar von dem jeweiligen Speichermedium gelesen wird, aber auf dem Speichermedium verbleibt und nur während des Lesens bzw. während der Darstellung und Bearbeitung temporär auf dem Computer vorhanden ist. Entsprechend greift bei der Erfindung der Computer 12 mit seiner Darstellungs-Software auf die Daten auf dem Kommunikations-Adapter 6 zu, die Daten verbleiben aber auf dem Kommunikations-Adapter 6.

Eine hierfür besonders geeignete Darstellungs-Software sind Internet-Browser, da sie sehr verbreitet, d.h. üblicherweise standardmäßig auf einem Computer installiert sind, standardisierte Übertragungsprotokolle und Datenformate verwenden und durch Sicherheitsstandards vorgegebene Gründe nur das Lesen von Daten, aber kein Schreiben von Daten gestatten bzw. keine Verändern, Löschen oder Überschreiben der gelesenen Daten gestatten, also Daten nur temporär zur Darstellung auf dem Computer 12 einlesen und keine Daten an den Kommunikations-Adapter 6 übertragen.

In manchen Ausführungsformen kann vorgesehen sein, dass der Benutzer an dem Computer 12 auch online mit einem Web-Server oder Host-Computer kommuniziert, beispielsweise auf einer Website des Herstellers seines Gerätes 1, und dieser Web-Server mit dem Kommunikations-Adapter 6 kommuniziert, der an den Computer 12 angeschlossen ist. Auf diese Weise kann beispielsweise ein Update der Soft- oder Firmware des Kommunikations-Adapters 6 und/oder des Gerätes 1 stattfinden.

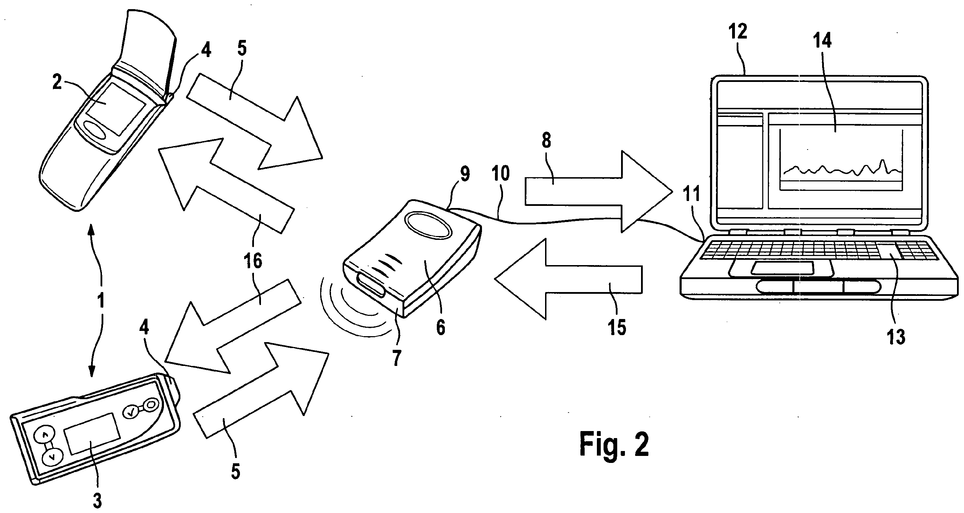

In Fig. 2 ist ein weiteres Ausführungsbeispiel dargestellt, bei dem eine bidirektionale Datenübertragung zur Anwendung kommt. Es entspricht demjenigen von Fig. 1, wobei eine weitere Funktionalität ausgebildet ist, nämlich die, dass der Kommunikations-Adapter 6 zum Übertragen von Daten an das Gerät 1 zum Steuern oder Programmieren des Geräts 1 von dem Computer 12 aus ausgebildet ist, wobei die Steuerung oder Programmierung des Geräts 1 von dem Computer 12 über die Computer-Schnittstelle 11 des Computers 12 an den Kommunikations-Adapter 6 und von dem Kommunikations-Adapter 6 über die Adapter-Geräte-Schnittstelle 7 des Kommunikations-Adapters 6 an das Gerät 1 erfolgt. Bei der in Fig. 2 dargestellten Ausführungsform findet also auch eine drahtgebundene Datenübertragung 15 von dem Computer 12 zu dem Kommunikations-Adapter 6 und eine Infrarot-Datenübertragung 16 von dem Kommunikations-Adapter 6 zu dem Gerät 1 statt.

In diesem Fall dient der Kommunikations-Adapter 6 sozusagen als "intelligentes Kabel" bzw. als USB-zu-IR-Adapter. Der Kommunikations-Adapter 6 setzt die Daten von dem USB-Protokoll in das IR-Protokoll und umgekehrt um. In diesem Fall kann auf dem Computer 12 eine zusätzliche spezielle Software mit den Gerätetreibern, z.B. für die Datenanalyse gespeichert werden, oder die Computer-Software nutzt die Gerätetreiber und die ausgelesenen Daten, die vom Kommunikations-Adapter 6 zur Verfügung gestellt werden. Für die tatsächliche Kommunikation zu dem Gerät 1 sorgt die Firmware in dem Kommunikations-Adapter 6. Die Daten können aus den Geräten 1 über die Datenübertragungen 5 und 8 ausgelesen und am Computer 12 dargestellt werden. Über die Datenübertragungen 15 und 16 können die Geräte 1 darüber hinaus bedient, programmiert oder eingestellt werden. Dadurch ist es beispielsweise möglich, bei einem Blutglukosemeter 2 Datum und Uhrzeit einzustellen oder bei einer Insulinpumpe 3 Datum und Uhrzeit einzustellen und ein bestimmtes zeitliches Basalratenprofil einzustellen.

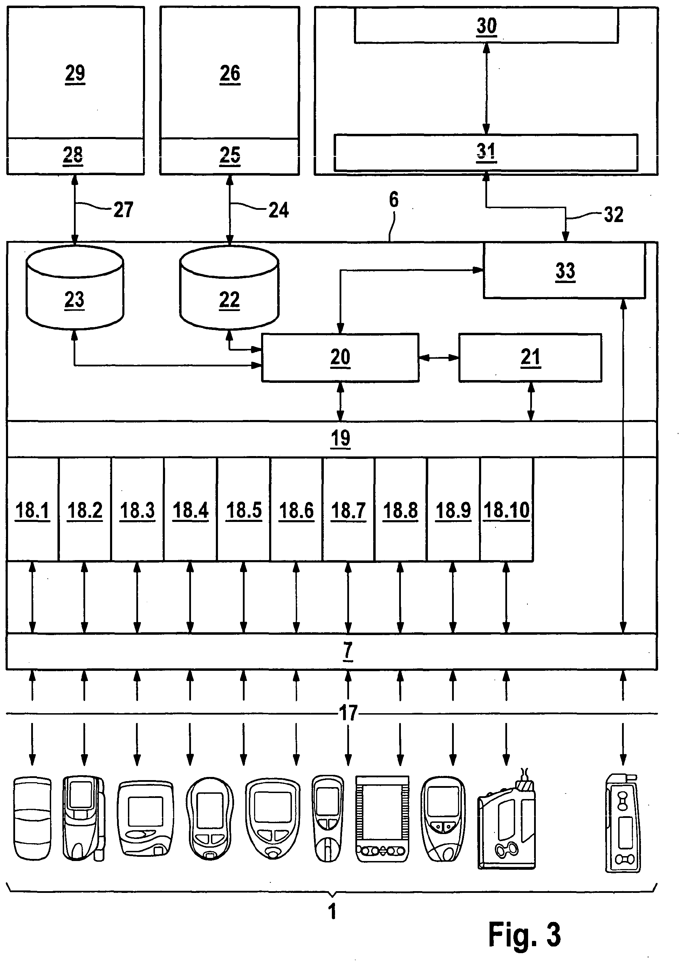

Die Figur 3 zeigt Details einer Implementierung eines Kommunikations-Adapters 6, an den zahlreiche verschiedene Geräte 1 angeschlossen werden können. Die Geräte 1 kommunizieren über gerätespezifische low level Geräteinterface 17 mit der Adapter-Geräte-Schnittstelle 7 des Kommunikations-Adapters 6. Der Kommunikations-Adapter 6 weist eine Mehrzahl von Gerätetreibern auf, die mittels unterschiedlicher Protokolle 18.1 bis 18.10 die geräteangepasste Kommunikation mit den Geräten 1 ermöglichen. Der universelle Dateimanager 19 steuert die Datenverarbeitung 20 des Prozessors und der Datenspeicher 21 in dem Kommunikations-Adapter 6. Die Datenausgabe erfolgt durch HTML-Berichte 22 oder in Form von XML-Daten 23. Die HTML-Berichte 22 werden über Lesebefehle 24 und den Datenzugriff 25 auf einem Internet-Browser 26 eines Computers 12 dargestellt. Die XML-Daten 23 werden über einen Lesebefehl 27 und einen Datenzugriff 28 anderen PC-Anwendungen, z.B. Excel, des Computers 12 bereitgestellt.

Daneben gibt es noch eine konsolidierte Funktionalität 30 eines Human Interface Device Treibers 31. Dieser erzeugt Lese- und Schreibbefehle 32 in Verbindung mit einem USB-IR-Kommando-Umsetzer 33 für die Hardware-Steuerung und das Ausführen von Befehlen.

Ein Kommunikations-Adapter 6 bietet dem Benutzer u.a. folgende, weiter oben angesprochene Vorteile: (i) Die Datenauswertung der von einem Gerät 1 aufgenommenen Daten ist möglich, ohne hierzu über eine spezielle Software verfügen und diese installieren zu müssen. (ii) Es kann eine automatische Erkennung des Geräts 1 durch den Kommunikations-Adapter 6 erfolgen, wodurch die Bedienung vereinfacht ist. (iii) Der Kommunikations-Adapter 6 benötigt in der bevorzugten Ausführungsform einer drahtgebundenen Adapter-Computer-Schnittstelle 9 keine eigene Stromversorgung. (iv) In der ebenfalls bevorzugten Ausführungsform erfolgt die Befehlssteuerung des Kommunikations-Adapters 6 über das Auslesen von Triggerdateien auf dem Kommunikations-Adapter 6 über die Adapter-Computer-Schnittstelle 9 von dem Computer 12 aus.

Ein Kommunikations-Adapter 6 ermöglicht eine einfache und effiziente Kommunikation zwischen Geräten 1 auf der einen Seite, beispielsweise Messgeräte wie Blutglukosemessgeräte, Therapiegeräte oder Verabreichungsgeräte wie Insulin-Pumpen oder Insulin-Pens, und intelligenten Einrichtungen zur Darstellung und Auswertung auf der anderen Seite, wie beispielsweise Palm-Tops oder Computern. Er trägt damit bei der Behandlung von Glukosestoffwechselstörungen zu einem "Circle of Care" bei, der drei Kernbereiche einschließt, nämlich den Collect-and-Monitoring-Bereich der Messwert- und Datenerfassung (z.B. Blutglukosewerte, Nahrungsaufnahmen, Übungen, Fettwerte, HbA1c, Fuß- und Augenuntersuchungen, Verabreichung von Medikamenten, Blutdruck), den Analyze-and-Advice-Bereich der Analyse und Empfehlung (z.B. Bolusempfehlung, Berichterzeugung, Risiko- und Potentialberichte) und den Act-Bereich des Handeln und, bei Diabeteserkrankungen, der Insulinverabreichung, einschließlich einer Optimierung der Therapie. Bei Diabeteserkrankungen umfasst dies Blutglukose-Messgeräte, Datenverarbeitungsgeräte wie den Kommunikations-Adapter 6 und Insulinverabreichungseinrichtungen.

### Bezugszeichenliste

- 1: Gerät
- 2: Blutglukosemeter
- 3: Insulinpumpe
- 4: Geräte-Adapter-Schnittstelle
- 5: IR-Datenübertragung
- 6: Kommunikations-Adapter
- 7: Adapater-Geräte-Schnittstelle
- 8: drahtgebundene Datenübertragung
- 9: Adapter-Computer-Schnittstelle
- 10: Übertragungskabel
- 11: Computer-Schnittstelle
- 12: Computer
- 13: Tastatur
- 14: Monitor
- 15: drahtgebundene Datenübertragung
- 16: IR-Datenübertragung
- 17: Geräteinterface
- 18: Protokoll
- 19: universeller Dateimanager
- 20: Datenverarbeitung
- 21: Datenspeicher
- 22: HTML-Bericht
- 23: XML-Daten
- 24: Lesebefehl
- 25: Datenzugriff
- 26: Internet-Browser
- 27: Lesebefehl
- 28: Datenzugriff
- 29: PC-Anwendungen
- 30: konsolidierte Funktionalität
- 31: Human Interface Device Treiber
- 32: Lese- und Schreibbefehl
- 33: Kommandoumsetzer

## Patentansprüche

1. Kommunikations-Adapter (6) zur Verwendung mit einem tragbaren, ambulant betreibbaren medizinischen oder therapeutischen Gerät (1), insbesondere einem Gerät (1) für die Diagnose oder Behandlung einer Glukosestoffwechselstörung, zum Übertragen von Daten zwischen dem medizinischen oder therapeutischen Gerät und einem Computer (12), der zum Anzeigen von Betriebsparametern oder Messdaten des Geräts (1) und/oder zum Bedienen des Geräts (1) dient,
wobei das medizinische oder therapeutische Gerät (1) umfasst
- einen Geräteprozessor zum Steuern des Gerätes (1) und
- eine Geräte-Adapter-Schnittstelle (4) zur Kommunikation des Geräteprozessors mit dem Kommunikations-Adapter (6),
und wobei der Kommunikations-Adapter (6) umfasst
- einen Adapterprozessor zum Steuern des Kommunikations-Adapters (6),
- eine Adapter-Geräte-Schnittstelle (7) zur Kommunikation des Kommunikations-Adapters (6) mit dem Gerät (1),
- eine Adapter-Computer-Schnittstelle (9) zur Kommunikation des Adapterprozessors mit einer Computer-Schnittstelle (11) des Computers (12) und
- einen Gerätetreiber mit zugehörigem Übertragungsprotokoll (18) für die Kommunikation mit dem Gerät (1),
**dadurch gekennzeichnet, dass**
der Kommunikations-Adapter (6) derart ausgebildet ist, dass er von dem Computer (12) aus dadurch steuerbar ist, dass von dem Computer (12) Lesebefehle an den Kommunikations-Adapter (6) gesendet werden, durch die in dem Kommunikations-Adapter (6) Triggerdateien gelesen werden, und das Lesen einer solchen Triggerdatei von der Soft- oder Firmware des Kommunikations-Adapters (6) als Steuerbefehl für den Kommunikations-Adapter (6) interpretiert wird.

2. Kommunikations-Adapter (6) nach Anspruch 1, **dadurch gekennzeichnet, dass** der Kommunikations-Adapter (6) die erforderliche Soft- oder Firmware umfasst, um die von dem Gerät (1) ausgelesenen Daten unabhängig von dem Computer (12) aufzubereiten und in einem von dem Computer (12) mit einem Internet-Browser darstellbaren Datenformat zum Auslesen durch den Computer (12) über die Adapter-Computer-Schnittstelle (9) für die Darstellung der Daten an dem Computer (12) bereitzustellen.

3. Kommunikations-Adapter (6) nach einem der vorhergehenden Ansprüche, **dadurch gekennzeichnet, dass** die Adapter-Geräte-Schnittstelle (7) zur Kommunikation des Kommunikations-Adapters (6) mit dem Gerät (1) eine Schnittstelle zur drahtlosen Datenübertragung, insbesondere eine Infrarot-Schnittstelle oder eine Funk-Schnittstelle, beispielsweise eine Bluetooth-Schnittstelle ist.

4. Kommunikations-Adapter (6) nach einem der vorhergehenden Ansprüche, **dadurch gekennzeichnet, dass** die Adapter-Computer-Schnittstelle (9) zur Kommunikation des Adapterprozessors mit der Computer-Schnittstelle (11) des Computers (12) eine Schnittstelle zur drahtgebundenen Datenübertragung ist.

5. Kommunikations-Adapter (6) nach dem vorhergehenden Anspruch, **dadurch gekennzeichnet, dass** die drahtgebundene Adapter-Computer-Schnittstelle (9) eine serielle Schnittstelle, eine parallele Schnittstelle, eine Firewire-Schnittstelle oder bevorzugt eine USB-Schnittstelle ist.

6. Kommunikations-Adapter (6) nach einem der vorhergehenden Ansprüche, **dadurch gekennzeichnet, dass** er eine Mehrzahl von Gerätetreibern mit zugehörigen Übertragungsprotokollen (18) aufweist, die bei einem über die Adapter-Geräte-Schnittstelle (7) mit dem Kommunikations-Adapter (6) kommunizierenden Gerät (1) automatisch abgefragt werden, um einen für das aktuelle Gerät (1) passenden Gerätetreiber aus der Mehrzahl der Gerätetreiber zu wählen.

7. Kommunikations-Adapter (6) nach einem der vorhergehenden Ansprüche, **dadurch gekennzeichnet, dass** der Kommunikations-Adapter (6) zusätzlich die von dem Gerät (1) ausgelesenen Daten in einem abspeicherbaren Datenformat erzeugt und diese Daten in mindestens einer Datei in dem Kommunikations-Adapter abspeichert, von wo sie über die Adapter-Computer-Schnittstelle (9) von dem Computer (12) auslesbar sind.

8. Kommunikations-Adapter (6) nach einem der vorhergehenden Ansprüche, **dadurch gekennzeichnet, dass** er keine eigene oder interne Stromquelle aufweist und seine Stromversorgung über die Adapter-Computer-Schnittstelle (9) von dem Computer (12) erfolgt.

9. Kommunikations-Adapter (6) nach einem der vorhergehenden Ansprüche, **dadurch gekennzeichnet, dass** er zum Programmieren des Geräts (1) von dem Computer (12) aus ausgebildet ist, wobei die Programmierung des Geräts (1) von dem Computer (12) über die Computer-Schnittstelle (11) des Computers (12) an den Kommunikations-Adapter (6) und von dem Kommunikations-Adapter (6) über die Adapter-Geräte-Schnittstelle des Kommunikations-Adapters (6) an das Gerät (1) erfolgt.

10. Kommunikations-Adapter (6) nach einem der vorhergehenden Ansprüche, **dadurch gekennzeichnet, dass** das Gerät (1) ein analytisches Messgerät zum Bestimmen oder Aufzeichnen eines medizinisch bedeutsamen Parameters, insbesondere ein Blutglukosemeter (2), ein Cholesterinmessgerät, ein Blutgerinnungsparameter-Messgerät, ein Blutdruckmessgerät, ein Gerät zum Messen des Körpergewichts, ein Blutglukoserecorder oder ein Blutdruckrecorder, oder ein Gerät zum Injizieren eines medizinischen Wirkstoffs in einen Körper, insbesondere eine Insulinpumpe (3), ist.

11. Kommunikations-Adapter (6) nach einem der vorausgehenden Ansprüche, **dadurch gekennzeichnet, dass** das Gerät (1) und der Kommunikations-Adapter (6) zum Übertragen von Betriebsdaten oder Messwerten des Geräts (1) von dem Gerät (1) mittels des Kommunikations-Adapters (6) an den Computer (12) ausgebildet sind, bei denen es sich um Betriebsdaten oder Messwerte handelt, die sich auf zurückliegende Zeitpunkte beziehen.

12. Verfahren zum Übertragen von Daten zwischen einem tragbaren, ambulant betreibbaren medizinischen oder therapeutischen Gerät (1), insbesondere einem Gerät (1) für die Diagnose oder Behandlung einer Glukosestoffwechselstörung und einem Computer (12), der zum Anzeigen von Betriebsparametern oder Messdaten des Geräts (1) und/oder zum Bedienen des Geräts (1) dient, mittels eines Kommunikations-Adapters (6),
wobei das medizinische oder therapeutische Gerät (1) umfasst
- einen Geräteprozessor, der das Gerät (1) steuert, und
- eine Geräte-Adapter-Schnittstelle (4) zur Kommunikation des Geräteprozessors mit dem Kommunikations-Adapter (6),
und wobei der Kommunikations-Adapter (6) umfasst
- einen Adapterprozessor, der den Kommunikations-Adapter (6) steuert,
- eine Adapter-Geräte-Schnittstelle (7) zur Kommunikation des Kommunikations-Adapters (6) mit dem Gerät (1),
- eine Adapter-Computer-Schnittstelle (9) zur Kommunikation des Adapterprozessors mit einer Computer-Schnittstelle (11) des Computers (12) und
- einen Gerätetreiber mit zugehörigem Übertragungsprotokoll (18) für die Kommunikation mit dem Gerät (1),
**dadurch gekennzeichnet, dass**
der Kommunikations-Adapter (6) von dem Computer (12) aus dadurch gesteuert wird, dass von dem Computer (12) Lesebefehle an den Kommunikations-Adapter (6) gesendet werden, durch die in dem Kommunikations-Adapter (6) Triggerdateien gelesen werden, und das Lesen einer solchen Triggerdatei von der Soft- oder Firmware des Kommunikations-Adapters (6) als Steuerbefehl für den Kommunikations-Adapter (6) interpretiert wird.

13. Verfahren nach Anspruch 12, **dadurch gekennzeichnet, dass** der Kommunikations-Adapter (6) die erforderliche Soft- oder Firmware umfasst, mit der die von dem Gerät (1) ausgelesenen Daten unabhängig von dem Computer (12) aufbereitet und in einem von dem Computer (12) mit einem Internet-Browser darstellbaren Datenformat zum Auslesen durch den Computer (12) über die Adapter-Computer-Schnittstelle (9) für die Darstellung der Daten an dem Computer (12) bereitgestellt werden.

14. Verfahren nach Anspruch 12 oder 13, **dadurch gekennzeichnet, dass** der Kommunikations-Adapter (6) eine Mehrzahl von Gerätetreibern mit zugehörigen Übertragungsprotokollen (18) aufweist, die bei einem über die Adapter-Geräte-Schnittstelle (7) mit dem Kommunikations-Adapter (6) kommunizierenden Gerät (1) automatisch abgefragt werden, um einen für das aktuelle Gerät (1) passenden Gerätetreiber aus der Mehrzahl der Gerätetreiber zu wählen.

15. System zum Betreiben eines tragbaren, ambulant betreibbaren medizinischen oder therapeutischen Geräts (1), insbesondere einem Gerät (1) für die Diagnose oder Behandlung einer Glukosestoffwechselstörung, mit einer Datenübertragung zwischen dem medizinischen oder therapeutischen Gerät (1) und einem Computer (12), der zum Anzeigen von Betriebsparametern oder Messdaten des Geräts (1) und/oder zum Bedienen des Geräts (1) dient, **dadurch gekennzeichnet, dass** es einen Kommunikations-Adapter (6) nach einem der Ansprüche 1 bis 11 und mindestens ein medizinisches oder therapeutisches Gerät (1) mit einem Geräteprozessor, der das Gerät (1) steuert und mit einer Geräte-Adapter-Schnittstelle (4) zur Kommunikation des Geräteprozessors mit dem Kommunikations-Adapter (6) umfasst.

16. System nach Anspruch 15, **dadurch gekennzeichnet, dass** das medizinische oder therapeutische Gerät (1) ein eigenständig, d.h. unabhängig von anderen angeschlossenen Komponenten oder Geräten, insbesondere ein ohne den Kommunikations-Adapter (6) betreibbares Gerät ist.

17. System nach Anspruch 15 oder 16, **dadurch gekennzeichnet, dass** das medizinische oder therapeutische Gerät (1) ein analytisches Messgerät zum Bestimmen oder Aufzeichnen eines medizinisch bedeutsamen Parameters, insbesondere ein Gerät für die Diagnose oder Behandlung von Glukosestoffwechselstörungen, insbesondere ein Blutglukosemeter (2), ein Blutzuckermessgerät oder ein Blutglukoserecorder, ein Cholesterinmessgerät, ein Gerät für die Messung eines Blutgerinnungsparameters, ein Blutdruckmessgerät, ein Blutdruckrecorder, ein Gerät zum Messen des Körpergewichts oder ein Gerät zum Injizieren eines medizinischen Wirkstoffs in einen Körper, insbesondere eine Insulinpumpe (3), ist.

18. System nach einem der Ansprüche 15 bis 17, **dadurch gekennzeichnet, dass** die Darstellungs-Software eine Software ist, die die darzustellenden Daten zwar von dem Kommunikations-Adapter (6) lesen, aber nicht auf dem Kommunikations-Adapter (6) bzw. in dem Gerät (1) ändern kann, so dass die mit dem Computer (12) von dem Gerät (1) ausgelesenen Daten nur zur temporären Darstellung an den Computer (12) übertragen werden, indem die Darstellungs-Software die Daten von dem Gerät (1) ausliest, die Darstellungs-Software aber die dabei auf dem Kommunikations-Adapter (6) verbleibenden Daten auf dem Kommunikations-Adapter (6) bzw. die auf dem Gerät (1) verbleibenden Daten auf dem Gerät (1) nicht löschen oder verändern kann.

## Claims

1. A communication adaptor (6) for use with a portable, outpatient-operable medical or therapeutic device (1), in particular a device (1) for the diagnosis or treatment of a glucose metabolism disorder, for transmitting data between the medical or therapeutic device and a computer (12) that is used for displaying operating parameters or measurement data of the device (1) and/or for operating the device (1),
wherein the medical or therapeutic device (1 comprises
- a device processor for controlling the device (1) and
- a device-adaptor interface (4) for enabling the device processor to communicate with the communication adaptor (6),
and wherein the communication adaptor (6) comprises
- an adaptor processor for controlling the communication adaptor (6),
- an adaptor-device interface (7) for enabling the communication adaptor (6) to communicate with the device (1),
- an adaptor-computer interface (9) for enabling the adaptor processor to communicate with a computer interface (11) of the computer (12) and
- a device driver with an allocated transmission protocol (18) for communication with the device (1),
**characterized in that**
the communication adaptor (6) is configured in such a way that it can be controlled from the computer (12) **in that** read commands, through which trigger files in the communication adaptor (6) are read, are sent by the computer (12) to the communication adaptor (6), and the reading of such a trigger file by the software or firmware of the communication adaptor (6) is interpreted as a control command for the communication adaptor (6).

2. The communication adaptor (6) according to claim 1, **characterized in that** the communication adaptor (6) comprises the necessary software or firmware for processing the data read from the device (1) independently of the computer (12) and converting to a data format displayable by the computer (12) with a web browser for readout by the computer (12) via the adaptor-computer interface (9) for the displaying of the data on the computer (12).

3. The communication adaptor (6) according to one of the preceding claims, **characterized in that** the adaptor-device interface (7) for enabling the communication adaptor (6) to communicate with the device (1) is an interface for wireless data transmission, in particular an infrared interface or a radio interface, for example a Bluetooth interface.

4. The communication adaptor (6) according to one of the preceding claims, **characterized in that** the adaptor-computer interface (9) for enabling the adaptor processor to communicate with the computer interface (11) of the computer (12) is an interface for wireline data transmission.

5. The communication adaptor (6) according to the preceding claim, **characterized in that** the wireline adaptor-computer interface (9) is a serial port, a parallel port, a FireWire port, or preferably a USB port.

6. The communication adaptor (6) according to one of the preceding claims, **characterized in that** it has a plurality of device drivers with allocated transmission protocols (18) which, in the case of a device (1) communicating with the communication adaptor (6) via the adaptor-device interface (7), are automatically queried in order to select a suitable device driver for the current device (1) from the plurality of device drivers.

7. The communication adaptor (6) according to one of the preceding claims, **characterized in that** said communication adaptor (6) in addition generates the data read from the device (1) in a saveable data format and saves these data in at least one file in the communication adaptor, from where they are readable by the computer (12) via the adaptor-computer interface (9).

8. The communication adaptor (6) according to one of the preceding claims, **characterized in that** it does not have an independent or internal power source and is supplied with power from the computer (12) via the adaptor-computer interface (9).

9. The communication adaptor (6) according to one of the preceding claims, **characterized in that** it is configured for programming the device (1) from the computer (12), wherein the device (1) is programmed on the communication adaptor (6) by the computer (12) via the computer interface (11) of the computer (12), and on the device (1) by the communication adaptor (6) via the adaptor-device interface of the communication adaptor (6).

10. The communication adaptor (6) according to one of the preceding claims, **characterized in that** the device (1) is an analytical measuring device for determining or recording a medically relevant parameter, in particular a blood glucose meter (2), a cholesterol measuring device, a blood coagulation parameter measuring device, a blood pressure measuring device, a device for measuring body weight, a blood glucose recorder or a blood pressure recorder, or a device for injecting a medicinal product into a body, in particular an insulin pump (3).

11. The communication adaptor (6) according to one of the preceding claims, **characterized in that** the device (1) and the communication adaptor (6) are configured for transmitting operational data or measurement values of the device (1) from the device (1) to the computer (12) by means of the communication adaptor (6), said operational data or measurement values relating to previous points in time.

12. A method of transmitting data between a portable, outpatient-operable medical or therapeutic device (1), in particular a device (1) for the diagnosis or treatment of a glucose metabolism disorder, and a computer (12) that is used for displaying operating parameters or measurement data of the device (1) and/or for operating the device (1), by means of a communication adaptor (6),
wherein the medical or therapeutic device (1) comprises
- a device processor, which controls the device (1), and
- a device-adaptor interface (4) for enabling the device processor to communicate with the communication adaptor (6),
and wherein the communication adaptor (6) comprises
- an adaptor processor, which controls the communication adaptor (6),
- an adaptor-device interface (7) for enabling the communication adaptor (6) to communicate with the device (1),
- an adaptor-computer interface (9) for enabling the adaptor processor to communicate with a computer interface (11) of the computers (12) and
- a device driver with an allocated transmission protocol (18) for communicating with the device (1),
**characterized in that**
the communications adaptor (6) is controlled from the computer (12) **in that** read commands, through which trigger files in the communication adaptor (6) are read, are sent by the computer (12) to the communication adaptor (6), and the reading of such a trigger file by the software or firmware of the communication adaptor (6) is interpreted as a control command for the communication adaptor (6).

13. The method according to claim 12, **characterized in that** the communication adaptor (6) comprises the necessary software or firmware with which the data read from the device (1) are processed independently of the computer (12) and converted to a data format displayable by the computer (12) with an internet browser for readout by the computer (12) via the adaptor-computer interface (9) for displaying the data on the computer (12).

14. The method according to claim 12 or claim 13, **characterized in that** the communication adaptor (6) has a plurality of device drivers with allocated transmission protocols (18) which, in the case of a device (1) communicating with the communication adaptor (6) via the adaptor-device interface (7), are automatically queried in order to select a suitable device driver for the current device (1) from the plurality of device drivers.

15. A system for operating a portable, outpatient-operable medical or therapeutic device (1), in particular a device (1) for the diagnosis or treatment of a glucose metabolism disorder, with a data transmission between the medical or therapeutic device (1) and a computer (12), which is used for displaying operating parameters and/or measurement data of the device (1) and/or for operating the device (1), **characterized in that** it comprises a communication adaptor (6) according to one of claims 1 to 11 and at least one medical or therapeutic device (1) with a device processor, which controls the device (1), and with a device-adaptor interface (4) for enabling the device processor to communicate with the communication adaptor (6).

16. The system according to claim 15, **characterized in that** the medical or therapeutic device (1) is a stand-alone device, i.e. one that is independent from other connected components or devices, in particular one that can be operated without the communication adaptor (6).

17. The system according to claim 15 or claim 16, **characterized in that** the medical or therapeutic device (1) is an analytical measuring device for determining or recording a medically relevant parameter, in particular a device for the diagnosis or treatment of glucose metabolism disorders, in particular a blood glucose meter (2), a blood sugar measuring device or a blood glucose recorder, a cholesterol measuring device, a device for measuring a blood coagulation parameter, a blood pressure measuring device, a blood pressure recorder, a device for measuring body weight, or a device for injecting a medicinal product into a body, in particular an insulin pump (3).

18. The system according to one of claims 15 to 17, **characterized in that** the imaging software is a software that can read the data to be displayed from the communication adaptor (6), but cannot alter them on the communication adaptor (6) or in the device (1) such that the data read from the device (1) with the computer (12) are transmitted to the computer (12) only for temporary display, wherein the imaging software reads the data from the device (1), but can neither delete nor alter the data remaining on the communication adaptor (6) on said communication adaptor (6) or the data remaining on the device (1) on said device (1) in this process.

## Revendications

1. Adaptateur de communication (6) destiné à être utilisé avec un dispositif médical ou thérapeutique (1) portable, utilisable en ambulatoire, en particulier un dispositif (1) pour le diagnostic ou le traitement d'un trouble du métabolisme du glucose, pour le transfert de données entre le dispositif médical ou thérapeutique et un ordinateur (12), qui sert à l'affichage de paramètres d'exploitation ou de données de mesure du dispositif (1) et/ou à l'utilisation du dispositif (1),
le dispositif médical ou thérapeutique (1) comprenant
- un processeur de dispositif pour commander le dispositif (1) et
- une interface dispositif-adaptateur (4) pour la communication du processeur de dispositif avec l'adaptateur de communication (6),
et l'adaptateur de communication (6) comprenant
- un processeur d'adaptateur pour commander l'adaptateur de communication (6),
- une interface adaptateur-dispositif (7), pour la communication de l'adaptateur de communication (6) avec le dispositif (1),
- une interface adaptateur-ordinateur (9) pour la communication du processeur d'adaptateur avec une interface d'ordinateur (11) de l'ordinateur (12) et
- un pilote, avec le protocole de transfert correspondant (18), pour la communication avec le dispositif (1),
**caractérisé en ce que**
l'adaptateur de communication (6) est configuré de telle sorte qu'il puisse être commandé par l'ordinateur (12) par le fait que des instructions de lecture sont envoyées par l'ordinateur (12) à l'adaptateur de communication (6), par lesquelles des fichiers de déclenchement sont lus dans l'adaptateur de communication (6), et la lecture d'un tel fichier de déclenchement est interprétée par le logiciel ou le microprogramme de l'adaptateur de communication (6) comme étant une instruction de commande pour l'adaptateur de communication (6).

2. Adaptateur de communication (6) selon la revendication 1, **caractérisé en ce que** l'adaptateur de communication (6) comprend le logiciel ou le microprogramme nécessaire pour préparer, indépendamment de l'ordinateur (12), les données lues par le dispositif (1), et les mettre à disposition, selon un format de données pouvant être représenté par l'ordinateur (12) avec un navigateur Internet, pour être lues par l'ordinateur (12) par l'intermédiaire de l'interface adaptateur-ordinateur (9), pour la représentation des données sur l'ordinateur (12).

3. Adaptateur de communication (6) selon l'une des revendications précédentes, **caractérisé en ce que** l'interface adaptateur-dispositif (7), pour la communication de l'adaptateur de communication (6) avec le dispositif (1), est une interface pour le transfert de données sans fil, en particulier une interface infrarouge ou une interface radio, par exemple une interface Bluetooth.

4. Adaptateur de communication (6) selon l'une des revendications précédentes, **caractérisé en ce que** l'interface adaptateur-ordinateur (9), pour la communication du processeur d'adaptateur avec l'interface d'ordinateur (11) de l'ordinateur (12), est une interface pour la transmission de données filaire.

5. Adaptateur de communication (6) selon la revendication précédente, **caractérisé en ce que** l'interface adaptateur-ordinateur (9) filaire est une interface série, une interface parallèle, une interface FireWire ou de préférence une interface USB.

6. Adaptateur de communication (6) selon l'une des revendications précédentes, **caractérisé en ce qu'**il présente une pluralité de pilotes, avec les protocoles de transfert correspondants (18), qui sont automatiquement interrogés par un dispositif (1), qui par l'intermédiaire de l'interface adaptateur-dispositif (7) communiquent avec l'adaptateur de communication (6), pour choisir, parmi la pluralité de pilotes, un pilote convenant au dispositif (1) effectif.

7. Adaptateur de communication (6) selon l'une des revendications précédentes, **caractérisé en ce que** l'adaptateur de communication (6) produit en outre, selon un format de données stockable, des données lues par le dispositif (1), et stocke ces données dans au moins un fichier dans l'adaptateur de communication, à partir duquel elles peuvent être lues par l'ordinateur (12) par l'intermédiaire de l'interface adaptateur-ordinateur (9).

8. Adaptateur de communication (6) selon l'une des revendications précédentes, **caractérisé en ce qu'**il ne comprend pas de source d'alimentation électrique propre ou interne, et que son alimentation électrique est assurée par l'ordinateur (12) par l'intermédiaire de l'interface adaptateur-ordinateur (9).

9. Adaptateur de communication (6) selon l'une des revendications précédentes, **caractérisé en ce qu'**il est configuré pour la programmation du dispositif (1) par l'ordinateur (12), la programmation du dispositif (1) étant réalisée sur l'adaptateur de communication (6) par l'ordinateur (12) par l'intermédiaire de l'interface d'ordinateur (11) de l'ordinateur (12), et sur le dispositif (1) par l'adaptateur de communication (6) par l'intermédiaire de l'interface adaptateur-dispositif de l'adaptateur de communication (6).

10. Adaptateur de communication (6) selon l'une des revendications précédentes, **caractérisé en ce que** le dispositif (1) est un appareil de mesure analytique pour la détermination ou l'enregistrement d'un paramètre médicalement significatif, en particulier un glucomètre (2), un analyseur de cholestérol, un appareil de mesure des paramètres de coagulation, un tensiomètre, un appareil pour mesurer le poids corporel, un enregistreur de glycémie ou un enregistreur de tension artérielle, ou un dispositif pour l'injection d'un principe actif médical dans un organisme, en particulier une pompe à insuline (3).

11. Adaptateur de communication (6) selon l'une des revendications précédentes, **caractérisé en ce que** le dispositif (1) et l'adaptateur de communication (6) sont configurés pour le transfert de données d'exploitation ou de valeurs de mesure du dispositif (1), du dispositif (1) à l'ordinateur (12) au moyen de l'adaptateur de communication (6), pour lesquels il s'agit de données d'exploitation ou de valeurs de mesure qui se rapportent à des instants antérieurs.

12. Procédé pour le transfert de données entre un dispositif médical ou thérapeutique (1) portable, utilisable en ambulatoire, en particulier un dispositif (1) pour le diagnostic ou le traitement d'un trouble du métabolisme du glucose, et un ordinateur (12), qui sert à l'affichage de paramètres d'exploitation ou de données de mesure du dispositif (1) et/ou à l'utilisation du dispositif (1), à à l'aide d'un adaptateur de communication (6),
le dispositif médical ou thérapeutique (1) comprenant
- un processeur de dispositif pour commander le dispositif (1), et
- une interface dispositif-adaptateur (4) pour la communication du processeur de dispositif avec l'adaptateur de communication (6),
et l'adaptateur de communication (6) comprenant
- un processeur d'adaptateur pour commander l'adaptateur de communication (6),
- une interface adaptateur-dispositif (7) pour la communication de l'adaptateur de communication (6) avec le dispositif (1),
- une interface adaptateur-ordinateur (9) pour la communication du processeur d'adaptateur avec une interface d'ordinateur (11) de l'ordinateur (12) et
- un pilote, avec le protocole de transfert correspondant (18), pour la communication avec le dispositif (1),
**caractérisé en ce que**
l'adaptateur de communication (6) est commandé par l'ordinateur (12) par le fait que des instructions de lecture sont envoyées par l'ordinateur (12) à l'adaptateur de communication (6), par lesquelles des fichiers de déclenchement sont lus dans l'adaptateur de communication (6), et la lecture d'un tel fichier de déclenchement est interprétée par le logiciel ou le microprogramme de l'adaptateur de communication (6) comme étant une instruction de commande pour l'adaptateur de communication (6).

13. Procédé selon la revendication 12, **caractérisé en ce que** l'adaptateur de communication (6) comprend le logiciel ou le microprogramme nécessaire, à l'aide duquel les données lues par le dispositif (1) sont préparées par l'ordinateur (12) et sont mises à disposition, selon un format de données pouvant être représenté par un ordinateur (12) avec un navigateur Internet, pour la lecture par l'ordinateur (12) par l'intermédiaire de l'interface adaptateur-ordinateur (9) pour la représentation des données sur l'ordinateur (12).

14. Procédé selon la revendication 12 ou 13, **caractérisé en ce que** l'adaptateur de communication (6) présente une pluralité de pilotes, avec les protocoles de transfert correspondants (18), qui sont automatiquement interrogés par un dispositif (1), qui par l'intermédiaire de l'interface adaptateur-dispositif (7) communiquent avec l'adaptateur de communication (6), pour choisir, parmi la pluralité de pilotes, un pilote convenant au dispositif (1) effectif.

15. Système pour l'exploitation d'un dispositif médical ou thérapeutique (1) portable, utilisable en ambulatoire, en particulier un appareil (1) pour le diagnostic ou le traitement d'un trouble du métabolisme du glucose, avec un transfert de données entre un dispositif médical ou thérapeutique (1) et un ordinateur (12), qui sert à l'affichage de paramètres d'exploitation ou de données de mesure du dispositif (1) et/ou à l'utilisation du dispositif (1), **caractérisé en ce qu'**il comprend un adaptateur de communication (6) selon l'une des revendications 1 à 11 et au moins un dispositif médical ou thérapeutique (1) avec un processeur de dispositif qui commande le dispositif (1) et comprend une interface dispositif-adaptateur (4) pour la communication du processeur de dispositif avec l'adaptateur de communication (6).

16. Système selon la revendication 15, **caractérisé en ce que** le dispositif médical ou thérapeutique (1) est un dispositif autonome, c'est-à-dire indépendant d'autres composants ou dispositifs connectés, en particulier un dispositif utilisable sans l'adaptateur de communication (6).

17. Système selon la revendication 15 ou 16, **caractérisé en ce que** le dispositif médical ou thérapeutique (1) est un appareil de mesure analytique pour la détermination ou l'enregistrement d'un paramètre médicalement significatif, en particulier un dispositif pour le diagnostic ou le traitement de troubles du métabolisme du glucose, en particulier un glucomètre (2), un dispositif de mesure du taux de sucre dans le sang ou un enregistreur de glycémie, un analyseur de cholestérol, un appareil de mesure des paramètres de coagulation, un tensiomètre, un enregistreur de tension artérielle, un appareil pour mesurer le poids corporel ou un dispositif pour l'injection d'un principe actif médical dans un organisme, en particulier une pompe à insuline (3).

18. Système selon l'une des revendications 15 à 17, **caractérisé en ce que** le logiciel de représentation est un logiciel qui, bien que pouvant lire les données à représenter par l'adaptateur de communication (6), ne peut les modifier sur l'adaptateur de communication (6) ou dans le dispositif (1), de sorte que les données lues par le dispositif (1) avec l'ordinateur (12) ne sont transférées à l'ordinateur (12) que pour une représentation temporaire, par le fait que le logiciel de représentation lit les données par le dispositif (1), mais le logiciel de représentation ne peut effacer ou modifier sur l'adaptateur de communication (6) les données qui restent alors sur l'adaptateur de communication (6), ou sur le dispositif (1) les données qui restent sur le dispositif (1).
